# EUROPÄISCHE PATENTSCHRIFT

(11) **EP 1 076 636 B1**
(45) Veröffentlichungstag und Bekanntmachung des Hinweises auf die Patenterteilung: **09.07.2003**
(21) Anmeldenummer: 99923430.5
(22) Anmeldetag: 21.04.1999
(51) Int. Cl.: C07B 37/06, C07C 1/207

(54) **VERFAHREN ZUR HERSTELLUNG VON SUBSTITUIERTEN BENZOLEN**
METHOD FOR PRODUCING SUBSTITUTED BENZENES
PROCEDE DE PREPARATION DE BENZENES SUBSTITUES

(30) Priorität: 30.04.1998 DE 19819375
(43) Veröffentlichungstag der Anmeldung: 21.02.2001
(73) Patentinhaber: Bayer CropScience AG, 40789 Monheim (DE)
(72) Erfinder: LINKER, Torsten, D-97078 Würzburg (DE); VORNDRAN, Katja, D-97080 Würzburg (DE)
(86) Internationale Anmeldenummer: EP9902681
(87) Internationale Veröffentlichungsnummer: WO99057084

(56) Entgegenhaltungen:
- G. S. R SUBBA RAO: "Michael reactions of the anions generated by the metal-ammonia reduction of benzoic acids" JOURNAL OF THE CHEMICAL SOCIETY, CHEMICAL COMMUNICATIONS,1980, Seiten 315-316, XP002112054 LETCHWORTH GB
- P. SENECI: "Synthesis of 2,5- and 2,4-cyclohexadiene-1,3-dicarboxylates via reductive alkylation of isophthalates" SYNTHETIC COMMUNICATIONS, Bd. 27, Nr. 5, 1997, Seiten 795-809, XP002112055
- A. J. BIRCH: "Oxidative decarboxylation of dihydroaromatic acids with lead tetraacetate: a synthesis of olivetol dimethyl ether and of rosefuran" TETRAHEDRON LETTERS, Nr. 24, 1976, Seiten 2079-2082, XP002112056 OXFORD GB in der Anmeldung erwähnt
- J. SLOBBE: "Anodic decarboxylation of dihydroaromatic acids" JOURNAL OF THE CHEMICAL SOCIETY, CHEMICAL COMMUNICATIONS,1977, Seiten 82-83, XP002112057 LETCHWORTH GB in der Anmeldung erwähnt

## Beschreibung

Die vorliegende Erfindung betrifft ein neues Verfahren zur Herstellung von bekannten, substituierten Benzolen, die als Zwischenprodukte zur Synthese von zahlreichen Pharmazeutika oder agrochemischen Wirkstoffen verwendbar sind.

Es ist bereits bekannt geworden, daß man substituierte Benzole erhält, wenn man Cyclohexa-2,5-dien-1-carbonsäure-Derivate entweder elektrochemisch oder mit Hilfe von Bleitetra-acetat oxydativ decarboxyliert (vgl. Organic Reactions 42, 26 (1992), Tetrahedron Letters 1976, 2079-2082 und J. Chem. Soc. Chem. Comm. 1977, 82). Nachteilig an diesen Verfahren ist aber, daß sie für eine Produktion der gewünschten Stoffe in technischem Maßstab kaum geeignet sind. So ist z.B. für die Durchführung der Reaktion auf elektrochemischem Wege ein relativ hoher apparativer Aufwand erforderlich. Verwendet man Bleitetra-acetat als Oxidationsmittel, so fallen größere Mengen an bleihaltigem Material an, das entsorgt werden muß. Außerdem ist Bleitetra-acetat eine recht teure Reaktionskomponente.

Es wurde nun gefunden, daß sich substituierte Benzole der Formel 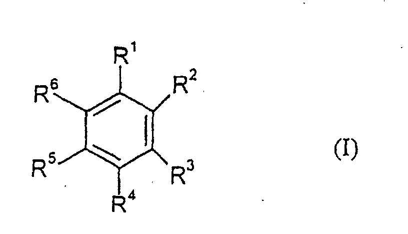 in welcher
- R¹: für gegebenenfalls durch Halogen, Hydroxy, Cyano, Carboxyl, Alkoxycarbonyl und/oder Alkylcarbonyl substituiertes Alkyl,
für gegebenenfalls durch Halogen, Hydroxy, Cyano, Carboxyl, Alkoxycarbonyl und/oder Alkylcarbonyl substituiertes Alkenyl,
für gegebenenfalls durch Halogen, Hydroxy, Cyano, Carboxyl, Alkoxycarbonyl und/oder Alkylcarbonyl substituiertes Alkinyl oder für gegebenenfalls durch Halogen, Hydroxy, Cyano, Carboxyl, Alkoxycarbonyl und/oder Alkylcarbonyl substituiertes Benzyl steht,
- R² und R³: gleich oder verschieden sind und für Wasserstoff, Fluor, Chlor, Alkyl, Phenyl oder Phenoxy stehen,
- R⁴: für Wasserstoff oder Alkyl steht und
- R⁵ und R⁶: gleich oder verschieden sind und für Wasserstoff, Fluor, Chlor, Alkyl, Phenyl oder Phenoxy stehen oder
- R⁵ und R⁶: gemeinsam für einen ankondensierten Benzolring stehen,
herstellen lassen, indem man Cxclohexa-2,5-dien-1-carbonsäure-Derivate der Formel 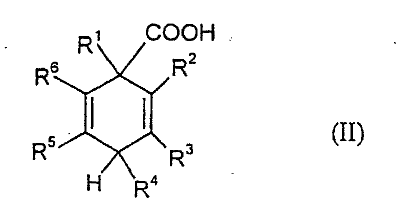 in welcher
- R¹, R², R³, R⁴, R⁵ und R⁶: die oben angegebenen Bedeutungen haben,
mit Säure gegebenenfalls in Gegenwart eines Verdünnungsmittels umsetzt.

Es ist als ausgesprochen überraschend zu bezeichnen, daß sich substituierte Benzole der Formel (I) nach dem erfindungsgemäßen Verfahren unter erheblich einfacheren Bedingungen herstellen lassen als nach den bisher bekannten Methoden, bei denen die gleichen Ausgangsprodukte eingesetzt werden. Unerwartet ist vor allem, daß die erfindungsgemäße Reaktion nach einem völlig anderen Mechanismus abläuft als die entsprechenden, zuvor beschriebenen oxidativen Decarboxylierungen. So entstehen die substituierten Benzole der Formel (I) im Falle des erfindungsgemäßen Verfahrens nicht durch Kohlendioxid-Abspaltung und Dehydrierung, sondern durch Abspaltung von Kohlenmonoxid und Wasser aus den Cyclohexa-2,5-dien-1-carbonsäure-Derivaten der Formel (II).

Das erfindungsgemäße Verfahren zeichnet sich durch eine Reihe von Vorteilen aus. So ermöglicht es die Synthese einer Vielzahl von teilweise kompliziert substituierten Benzolen in hoher Ausbeute und sehr guter Reinheit. Günstig ist auch, daß die benötigten Ausganssstoffe einfach herstellbar und auch in größeren Mengen verfügbar sind. Ein weiterer Vorteil besteht schließlich darin, daß als Reaktionskomponente nur Säure erforderlich ist und daß die Durchführung der Umsetzung sowie die Isolierung der Reaktionsprodukte keinerlei Schwierigkeiten bereitet.

Verwendet man 1-Isopropyl-2-methyl-cyclohexa-2,5-dien-1-carbonsäure als Ausgangssubstanz und setzt diese mit Chlorsulfonsäure um, so kann der Verlauf des erfindungsgemäßen Verfahrens durch das folgende Formelschema veranschaulicht werden.

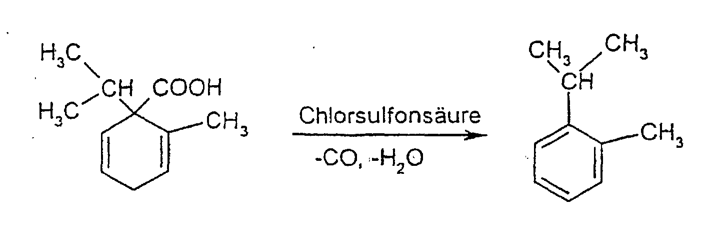

Die bei der Durchführung des erfindungsgemäßen Verfahrens als Ausgangsstoffe benötigten Cyclohexa-2,5-dien-1-carbonsäure-Derivate sind durch die Formel (II) allgemein definiert. Bevorzugt einsetzbar sind Verbindungen der Formel (II), in denen
- R¹: für geradkettiges oder verzweigtes Alkyl mit 1 bis 6 Kohlenstoffatomen steht, das einfach bis dreifach, gleichartig oder verschieden substituiert sein kann durch Fluor, Chlor, Hydroxy, Cyano, Carboxyl, Alkoxycarbonyl mit 1 bis 6 Kohlenstoffatomen in der Alkoxygruppe und/oder durch Alkylcarbonyl mit 1 bis 6 Kohlenstoffatomen im Alkylteil, oder
- R¹: für geradkettiges oder verzweigtes Alkenyl mit 2 bis 6 Kohlenstoffatomen steht, das einfach bis dreifach, gleichartig oder verschieden substituiert sein kann durch Fluor, Chlor, Hydroxy, Cyano, Carboxyl, Alkoxycarbonyl mit 1 bis 6 Kohlenstoffatomen in der Alkoxygruppe und/oder durch Alkylcarbonyl mit 1 bis 6 Kohlenstoffatomen im Alkylteil, oder
- R¹: für geradkettiges oder verzweigtes Alkinyl mit 2 bis 6 Kohlenstoffatomen steht, das einfach bis dreifach, gleichartig oder verschieden substituiert sein kann durch Fluor, Chlor, Hydroxy, Cyano, Carboxyl, Alkoxycarbonyl mit 1 bis 6 Kohlenstoffatomen in der Alkoxygruppe und/oder durch Alkylcarbonyl mit 1 bis 6 Kohlenstoffatomen im Alkylteil, oder
- R¹: für Benzyl steht, das einfach bis dreifach, gleichartig oder verschieden substituiert sein kann durch Fluor, Chlor, Hydroxy, Cyano, Carboxyl, Alkoxycarbonyl mit 1 bis 6 Kohlenstoffatomen in der Alkoxygruppe und/oder durch Alkylcarbonyl mit 1 bis 6 Kohlenstoffatomen im Alkylteil,
- R² und R³: gleich oder verschieden sind und für Wasserstoff, Fluor, Chlor, Alkyl mit 1 bis 6 Kohlenstoffatomen, Phenyl oder Phenoxy stehen,

- R⁴: für Wasserstoff oder Alkyl mit 1 bis 6 Kohlenstoffatomen steht und
- R⁵ und R⁶: gleich oder verschieden sind und für Wasserstoff, Fluor, Chlor, Alkyl mit 1 bis 6 Kohlenstoffatomen, Phenyl oder Phenoxy stehen oder
- R⁵ und R⁶: gemeinsam für einen ankondensierten Benzolring stehen.
- R¹: steht besonders bevorzugt für geradkettiges oder verzweigtes Alkyl mit 1 bis 4 Kohlenstoffatomen, das einfach bis dreifach, gleichartig oder verschieden substituiert sein kann durch Fluor, Chlor, Hydroxy, Cyano, Carboxyl. Alkoxycarbonyl mit 1 bis 4 Kohlenstoffatomen im Alkoxyteil und/oder durch Alkylcarbonyl mit 1 bis 4 Kohlenstoffatomen im Alkylteil.
- R¹: steht außerdem besonders bevorzugt für geradkettiges oder verzweigtes Alkenyl mit 2 bis 4 Kohlenstoffatomen, das einfach bis dreifach, gleichartig oder verschieden substituiert sein kann durch Fluor, Chlor, Hydroxy, Cyano, Carboxyl, Alkoxycarbonyl mit 1 bis 4 Kohlenstoffatomen im Alkoxyteil und/oder durch Alkylcarbonyl mit 1 bis 4 Kohlenstoffatomen im Alkylteil.
- R¹: steht außerdem besonders bevorzugt für geradkettiges oder verzweigtes Alkinyl mit 2 bis 4 Kohlenstoffatomen, das einfach bis dreifach, gleichartig oder verschieden substituiert sein kann durch Fluor, Chlor, Hydroxy, Cyano, Carboxyl, Alkoxycarbonyl mit 1 bis 4 Kohlenstoffatomen im Alkoxyteil und/oder durch Alkylcarbonyl mit 1 bis 4 Kohlenstoffatomen im Alkylteil.
- R¹: steht außerdem besonders bevorzugt für geradkettiges oder verzweigtes Benzyl, das einfach bis dreifach, gleichartig oder verschieden substituiert sein kann durch Fluor, Chlor, Hydroxy, Cyano, Carboxyl, Alkoxycarbonyl mit 1 bis 4 Kohlenstoffatomen im Alkoxyteil und/oder durch Alkylcarbonyl mit 1 bis 4 Kohlenstoffatomen im Alkylteil.

- R² und R³: stehen unabhängig voneinander besonders bevorzugt für Wasserstoff, Fluor, Chlor, Alkyl mit 1 bis 4 Kohlenstoffatomen, Phenyl oder Phenoxy.
- R⁴: steht besonders bevorzugt für Wasserstoff oder Alkyl mit 1 bis 4 Kohlenstoffatomen.
- R⁵ und R⁶: stehen unabhängig voneinander besonders bevorzugt für Wasserstoff, Fluor, Chlor, Alkyl mit 1 bis 4 Kohlenstoffatomen, Phenyl oder Phenoxy.
- R⁵ und R⁶: stehen außerdem gemeinsam besonders bevorzugt für einen ankondensierten Benzolring.
- R¹: steht ganz besonders bevorzugt für Methyl, Ethyl, n-Propyl, Isopropyl, n-Butyl, sek.-Butyl, iso-Butyl oder tert.-Butyl, wobei jeder dieser Reste einfach bis dreifach, gleichartig oder verschieden substituiert sein kann durch Fluor, Chlor, Hydroxy, Cyano, Carboxyl, Methoxycarbonyl, Ethoxycarbonyl, Methylcarbonyl und/oder Ethylcarbonyl.
- R¹: steht außderdem ganz besonders bevorzugt für Vinyl, Prop-1-enyl, Prop-2-enyl, But-1-enyl oder But-2-enyl, wobei jeder dieser Reste einfach bis dreifach, gleichartig oder verschieden substituiert sein kann durch Fluor, Chlor, Hydroxy, Cyano, Carboxyl, Methoxycarbonyl, Ethoxycarbonyl, Methylcarbonyl und/oder Ethylcarbonyl.
- R¹: steht außerdem ganz besonders bevorzugt für Propargyl, das einfach bis dreifach, gleichartig oder verschieden substituiert sein kann durch Fluor, Chlor, Hydroxy, Cyano, Carboxyl, Methoxycarbonyl, Ethoxycarbonyl, Methylcarbonyl und/oder Ethylcarbonyl.
- R¹: steht außerdem ganz besonders bevorzugt für Benzyl, das einfach bis dreifach, gleichartig oder verschieden substituiert sein kann durch Fluor, Chlor, Hydroxy, Cyano, Carboxyl, Methoxycarbonyl, Ethoxycarbonyl, Methylcarbonyl und/oder Ethylcarbonyl.
- R² und R³: stehen unabhängig voneinander ganz besonders bevorzugt für Wasserstoff, Fluor, Chlor, Methyl, Ethyl, n-Propyl, Isopropyl, n-Butyl, sek.-Butyl, iso-Butyl, tert.-Butyl, Phenyl oder Phenoxy.
- R⁴: steht ganz besonders bevorzugt für Wasserstoff, Methyl, Ethyl, n-Propyl, Isopropyl, n-Butyl, sek.-Butyl, iso-Butyl oder tert.-Butyl.
- R⁵ und R⁶: stehen unabhängig voneinander ganz besonders bevorzugt für Wasserstoff, Fluor, Chlor, Methyl, Ethyl, n-Propyl, Isopropyl, n-Butyl, sek.-Butyl, iso-Butyl, tert.-Butyl, Phenyl oder Phenoxy.
- R⁵ und R⁶: stehen außerdem gemeinsam ganz besonders bevorzugt für einen ankondensierten Benzolring.

Die angegebenen Substituenten-Definitionen sind untereinander beliebig kombinierbar. Außerdem können auch einzelne Substituenten-Definitionen entfallen.

Eine besonders bevorzugte Gruppe von Ausgangsstoffen sind Cyclohexa-2,5-dien-1-carbonsäure-Derivate der Formel 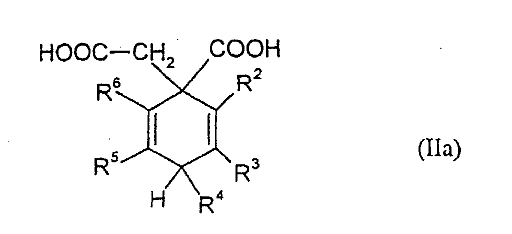 in welcher
- R², R³, R⁴, R⁵ und R⁶: diejenigen Bedeutungen haben, die oben als ganz besonders bevorzugt genannt wurden.

Eine weitere Gruppe von besonders bevorzugten Ausgangsstoffen sind Cyclohexa-2,5-dien-1-carbonsäure-Derivate der Formel 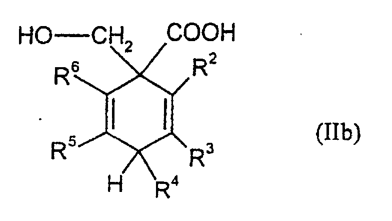 in welcher
- R², R³, R⁴, R⁵ und R⁶: diejenigen Bedeutungen haben, die oben als ganz besonders bevorzugt genannt wurden.

Eine weitere Gruppe von besonders bevorzugten Ausgangsstoffen sind Cyclohexa-2,5-dien-1-carbonsäure-Derivate der Formel 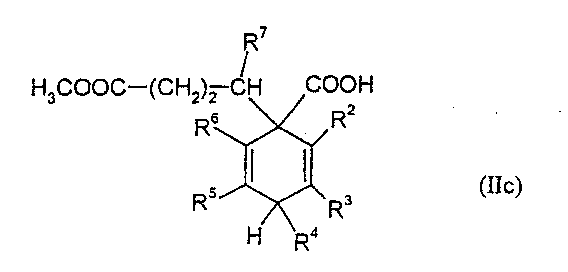 in welcher
- R², R³, R⁴, R⁵ und R⁶: diejenigen Bedeutungen haben, die oben als ganz besonders bevorzugt genannt wurden, und
- R⁷: für Wasserstoff oder Methyl steht.

Eine weitere Gruppe von besonders bevorzugten Ausgangsstoffen sind Cyclohexa-2,5-dien-1-carbonsäure-Derivate der Formel 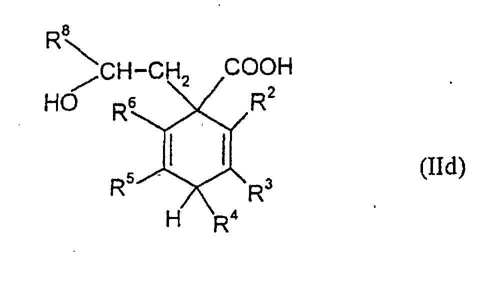 in welcher
- R², R³, R⁴, R⁵ und R⁶: diejenigen Bedeutungen haben, die oben als ganz besonders bevorzugt genannt wurden, und
- R⁸: für Wasserstoff, Methyl oder Ethyl steht.

Die Cyclohexa-2,5-dien-1-carbonsäure-Derivate der Formel (II) sind bekannt oder lassen sich nach bekannten Methoden herstellen (vgl. Organic Reactions 42, 23 ff (1992), Chem. Rev. 42, 24(1992), Chem. Ber. 94, 2095 (1961), Zh. Org. Khim. 1974 (10), 212, J. Amer. Chem. Soc. 1978, 100, 352, J. Chem. Soc. Chem. Comm. 1980, 315, Chem. Res. 42, 261-266 (1992) und Tetrahedron Letters 1985, 2039). So erhält man Cyclohexa-2,5-dien-1-carbonsäure-Derivate der Formel (II) z.B. aus entsprechenden Benzol- oder Naphthalincarbonsäuren durch Birch-Reduktion und gegebenenfalls nachfolgende Alkylierung.

Als Säuren kommen bei der Durchführung des erfindungsgemäßen Verfahrens alle üblichen starken, dehydratisierend wirkenden anorganischen oder organischen Säuren in Betracht. Vorzugsweise verwendbar sind Schwefelsäure und Chlorsulfonsäure.

Das erfindungsgemäße Verfahren wird vorzugsweise in Gegenwart eines Verdünnungsmittels durchgeführt.

Als Verdünnungsmittel kommen bei der Durchführung des erfindungsgemäßen Verfahrens alle üblichen, unter den Reaktionsbedingungen inerten organischen Solventien in Frage. Vorzugsweise verwendbar sind gegebenenfalls halogenierte aliphatische, cycloaliphatische oder aromatische Kohlenwasserstoffe, wie Pentan, Hexan, Cyclohexan, Methylcyclohexan, Benzol, Toluol, Chlorbenzol oder Methylenchlorid.

Die Reaktionstemperaturen können bei der Durchführung des erfindungsgemäßen Verfahrens innerhalb eines bestimmten Bereiches variiert werden. Im allgemeinen arbeitet man bei Temperaturen zwischen -30°C und +50°C, vorzugsweise zwischen -10°C und +25°C.

Bei der Durchführung des erfindungsgemäßen Verfahrens setzt man auf 1 mol an Cyclohexa-2,5-dien-1-carbonsäure-Derivat der Formel (II) eine äquivalente Menge oder auch einen geringen Überschuß an Säure ein.

Die Aufarbeitung erfolgt nach üblichen Methoden. Im allgemeinen verfährt man in der Weise, daß man das Reaktionsgemisch mit wäßrig-alkalischer Lösung versetzt und mehrfach mit einem mit Wasser wenig mischbaren organischen Lösungsmittel extrahiert, die vereinigten organischen Phasen trocknet und unter vermindertem Druck einengt. Die anfallenden Produkte können nach üblichen Methoden, z.B. durch Destillation oder auch Umkristailisation, von eventuell noch vorhandenen Verunreinigungen befreit werden.

Die nach dem erfindungsgemäßen Verfahren herstellbaren substituierten Benzole der Formel (I) sind wertvolle Zwischenprodukte zur Synthese von Wirkstoffen mit pharmazeutischen Eigenschaften oder von agrochemischen Wirkstoffen (vgl. EP-A 0 528 156).

So läßt sich z.B. die insektizid wirksame Verbindung der Formel 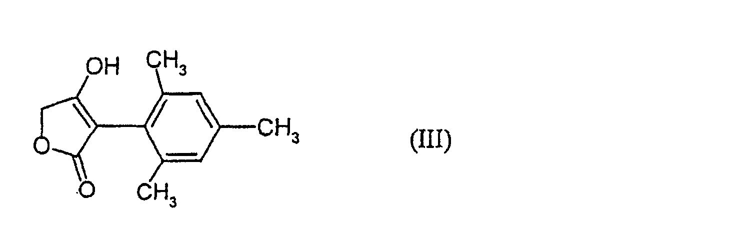 herstellen, indem man 2,4,6-Trimethyl-phenylessigsäure der Formel 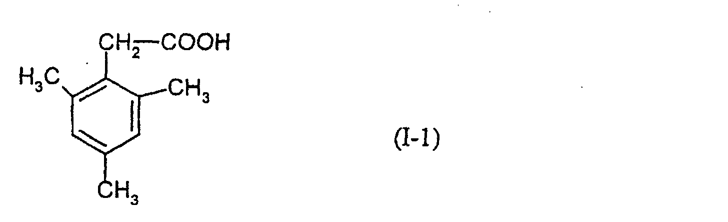 mit Bromessigsäureethylester in Gegenwart von tert.-Butanol und Kalium-tert.-butylat umsetzt und den dabei erhaltenen 2,4,6-Trimethylphenylessigsäure-ethoxycarbonylmethylester der Formel 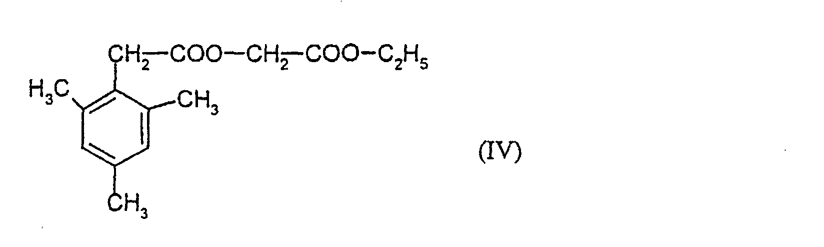 mit Kalium-tert.-butylat in Gegenwart von tert.-Butanol intramolekular cyclisiert.

Die Durchführung des erfindungsgemäßen Verfahrens wird durch die folgenden Beispiele veranschaulicht.

### Herstellungsbeispiele

### Beispiel 1

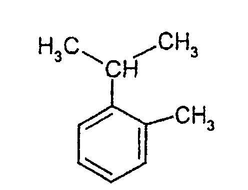

In eine Lösung von 1,8 g (10 mmol) 1-Isopropyl-2-methyl-cyclohexa-2,5-dien-1-carbonsäure in 20 ml trockenem Dichlormethan werden bei 0°C unter Rühren 1,16 g (10 mmol) Chlorsulfonsäure langsam eingetropft, wobei eine starke Gasentwicklung einsetzt. Nach beendeter Zugabe wird noch 10 Minuten bei 0°C nachgerührt. Anschließend wird das Reaktionsgemisch mit 20 ml gesättigter, wäßriger Natriumhydrogencarbonat-Lösung versetzt und viermal mit je 20 ml Dichlormethan extrahiert. Die vereinigten organischen Phasen werden über Natriumsulfat getrocknet und dann bei 35°C unter einem Druck von 700 mbar eingeengt. Man erhält auf diese Weise 1,27 g (95 % der Theorie) an 2-Isospropyl-toluol.

Nach der zuvor angegebenen Methode werden auch die in der folgenden Tabelle aufgeführten substituierten Benzole der Formel 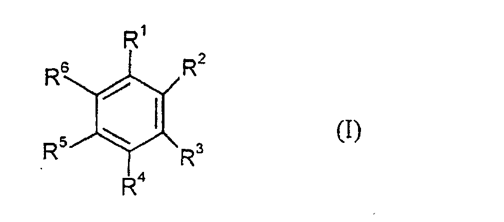 erhalten.

| **Beispiel Nr.** | **R**^{**1**} | **R**^{**2**} | **R**^{**3**} | **R**^{**4**} | **R**^{**5**} | **R**^{**6**} | **Ausbeute (%)** |
|---|---|---|---|---|---|---|---|
| 2 | CH₃ | CH₃ | H | H | H | H | 95 |
| 3 | C₂H₅ | CH₃ | H | H | H | H | 86 |
| 4 | C₄H₉ | CH₃ | H | H | H | H | 95 |
| 5 | C₂H₅ | H | H | CH₃ | H | H | 87 |
| 6 | (CH₃)₂CH | H | H | CH₃ | H | H | 93 |
| 7 | CH₂CO₂H | CH₃ | H | CH₃ | H | CH₃ | 89 |

## Patentansprüche

1. Verfahren zur Herstellung von substituierten Benzolen der Formel 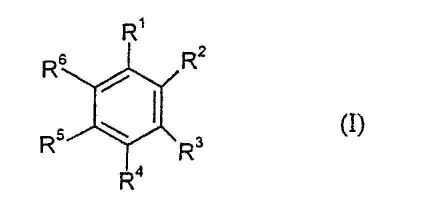 in welcher
R¹ für gegebenenfalls durch Halogen, Hydroxy, Cyano, Carboxyl, Alkoxycarbonyl und/oder Alkylcarbonyl substituiertes Alkyl, für gegebenenfalls durch Halogen, Hydroxy, Cyano, Carboxyl, Alkoxycarbonyl und/oder Alkylcarbonyl substituiertes Alkenyl, für gegebenenfalls durch Halogen, Hydroxy, Cyano, Carboxyl, Alkoxycarbonyl und/oder Alkylcarbonyl substituiertes Alkinyl oder für gegebenenfalls durch Halogen, Hydroxy, Cyano, Carboxyl, Alkoxycarbonyl und/oder Alkylcarbonyl substituiertes Benzyl steht,
R² und R³ gleich oder verschieden sind und für Wasserstoff, Fluor, Chlor, Alkyl, Phenyl oder Phenoxy stehen,
R⁴ für Wasserstoff oder Alkyl steht und
R⁵ und R⁶ gleich oder verschieden sind und für Wasserstoff, Fluor, Chlor, Alkyl, Phenyl oder Phenoxy stehen oder
R⁵ und R⁶ gemeinsam für einen ankondensierten Benzolring stehen, **dadurch gekennzeichnet, daß** man Cyclohexa-2,5-dien-1-carbonsäure-Derivate der Formel 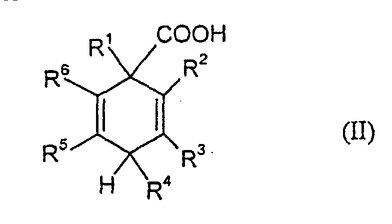
in welcher
R¹, R², R³, R⁴, R⁵ und R⁶ die oben angegebenen Bedeutungen haben,
mit Säure gegebenenfalls in Gegenwart eines Verdünnungsmittels umsetzt.

2. Verfahren gemäß Anspruch 1, **dadurch gekennzeichnet, daß** man als Ausgangsstoffe Cyclohexa-2,5-dien-1-carbonsäure-Derivate der Formel (II) einsetzt, in denen
R¹ für geradkettiges oder verzweigtes Alkyl mit 1 bis 6 Kohlenstoffatomen steht, das einfach bis dreifach, gleichartig oder verschieden substituiert sein kann durch Fluor, Chlor, Hydroxy, Cyano, Carboxyl, Alkoxycarbonyl mit 1 bis 6 Kohlenstoffatomen in der Alkoxygruppe und/oder durch Alkylcarbonyl mit 1 bis 6 Kohlenstoffatomen im Alkylteil, oder
R¹ für geradkettiges oder verzweigtes Alkenyl mit 2 bis 6 Kohlenstoffatomen steht, das einfach bis dreifach, gleichartig oder verschieden substituiert sein kann durch Fluor, Chlor, Hydroxy, Cyano, Carboxyl, Alkoxycarbonyl mit 1 bis 6 Kohlenstoffatomen in der Alkoxygruppe und/oder durch Alkylcarbonyl mit 1 bis 6 Kohlenstoffatomen im Alkylteil, oder
R¹ für geradkettiges oder verzweigtes Alkinyl mit 2 bis 6 Kohlenstoffatomen steht, das einfach bis dreifach, gleichartig oder verschieden substituiert sein kann durch Fluor, Chlor, Hydroxy, Cyano, Carboxyl, Alkoxycarbonyl mit 1 bis 6 Kohlenstoffatomen in der Alkoxygruppe und/oder durch Alkylcarbonyl mit 1 bis 6 Kohlenstoffatomen im Alkylteil, oder
R¹ für Benzyl steht, das einfach bis dreifach, gleichartig oder verschieden substituiert sein kann durch Fluor, Chlor, Hydroxy, Cyano, Carboxyl, Alkoxycarbonyl mit 1 bis 6 Kohlenstoffatomen in der Alkoxygruppe und/oder durch Alkylcarbonyl mit 1 bis 6 Kohlenstoffatomen im Alkylteil,
R² und R³ gleich oder verschieden sind und für Wasserstoff, Fluor, Chlor, Alkyl mit 1 bis 6 Kohlenstoffatomen, Phenyl oder Phenoxy stehen,
R⁴ für Wasserstoff oder Alkyl mit 1 bis 6 Kohlenstoffatomen steht und
R⁵ und R⁶ gleich oder verschieden sind und für Wasserstoff, Fluor, Chlor, Alkyl mit 1 bis 6 Kohlenstoffatomen, Phenyl oder Phenoxy stehen oder
R⁵ und R⁶ gemeinsam für einen ankondensierten Benzolring stehen.

3. Verfahren gemäß Anspruch 1, **dadurch gekennzeichnet, daß** man als Ausgangsstoffe Cyclohexa-2,5-dien-1-carbonsäure-Derivate der Formel (II) einsetzt, in denen
R¹ für geradkettiges oder verzweigtes Alkyl mit 1 bis 4 Kohlenstoffatomen steht, das einfach bis dreifach, gleichartig oder verschieden substituiert sein kann durch Fluor, Chlor, Hydroxy, Cyano, Carboxyl, Alkoxycarbonyl mit 1 bis 4 Kohlenstoffatomen im Alkoxyteil und/oder durch Alkylcarbonyl mit 1 bis 4 Kohlenstoffatomen im Alkylteil, oder
R¹ für geradkettiges oder verzweigtes Alkenyl mit 2 bis 4 Kohlenstoffatomen steht, das einfach bis dreifach, gleichartig oder verschieden substituiert sein kann durch Fluor, Chlor, Hydroxy, Cyano, Carboxyl, Alkoxycarbonyl mit 1 bis 4 Kohlenstoffatomen im Alkoxyteil und/oder durch Alkylcarbonyl mit 1 bis 4 Kohlenstoffatomen im Alkylteil, oder
R¹ für geradkettiges oder verzweigtes Alkinyl mit 2 bis 4 Kohlenstoffatomen steht, das einfach bis dreifach, gleichartig oder verschieden substituiert sein kann durch Fluor, Chlor, Hydroxy, Cyano, Carboxyl, Alkoxycarbonyl mit 1 bis 4 Kohlenstoffatomen im Alkoxyteil und/oder durch Alkylcarbonyl mit 1 bis 4 Kohlenstoffatomen im Alkylteil, oder
R¹ für Benzyl steht, das einfach bis dreifach, gleichartig oder verschieden substituiert sein kann durch Fluor, Chlor, Hydroxy, Cyano, Carboxyl, Alkoxycarbonyl mit 1 bis 4 Kohlenstoffatomen im Alkoxyteil und/oder durch Alkylcarbonyl mit 1 bis 4 Kohlenstoffatomen im Alkylteil,
R² und R³ unabhängig voneinander für Wasserstoff, Fluor, Chlor, Alkyl mit 1 bis 4 Kohlenstoffatomen, Phenyl oder Phenoxy stehen.
R⁴ für Wasserstoff oder Alkyl mit 1 bis 4 Kohlenstoffatomen steht und
R⁵ und R⁶ unabhängig voneinander für Wasserstoff, Fluor, Chlor, Alkyl mit 1 bis 4 Kohlenstoffatomen, Phenyl oder Phenoxy stehen oder
R⁵ und R⁶ gemeinsam für einen ankondensierten Benzolring stehen.

4. Verfahren gemäß Anspruch 1, **dadurch gekennzeichnet, daß** man Schwefelsäure oder Chlorsulfonsäure als Reaktionskomponente einsetzt.

5. Verfahren gemäß Anspruch 1, **dadurch gekennzeichnet, daß** man bei Temperaturen zwischen -30°C und +50°C arbeitet.

## Claims

1. Process for preparing substituted benzenes of the formula 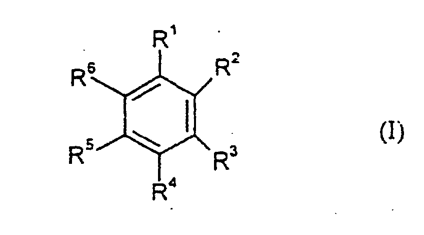 where
R¹ is alkyl which is optionally substituted by halogen, hydroxyl, cyano, carboxyl, alkoxycarbonyl and/or alkylcarbonyl, alkenyl which is optionally substituted by halogen, hydroxyl, cyano, carboxyl, alkoxycarbonyl and/or alkylcarbonyl, alkynyl which is optionally substituted by halogen, hydroxyl, cyano, carboxyl, alkoxycarbonyl and/or alkylcarbonyl, or benzyl which is optionally substituted by halogen, hydroxyl, cyano, carboxyl, alkoxycarbonyl and/or alkylcarbonyl,
R² and R³ are the same or different and are each hydrogen, fluorine, chlorine, alkyl, phenyl or phenoxy,
R⁴ is hydrogen or alkyl and
R⁵ and R⁶ are the same or different and are each hydrogen, fluorine, chlorine, alkyl, phenyl or phenoxy or
R⁵ and R⁶ together are a fused-on benzene ring,
**characterized in that** cyclohexa-2,5-diene-1-carboxylic acid derivatives of the formula 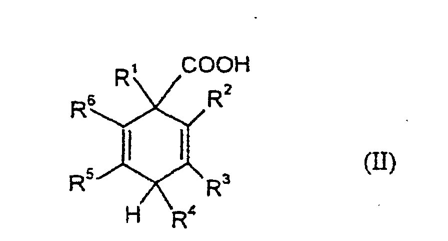 where
R¹, R², R³, R⁴, R⁵ and R⁶ are each as defined above
are reacted with acid, optionally in the presence of a diluent.

2. Process according to Claim 1, **characterized in that** the starting materials used are cyclohexa-2,5-diene-1-carboxylic acid derivatives of the formula (II) where
R¹ is straight-chain or branched alkyl having 1 to 6 carbon atoms which may be mono- to trisubstituted identically or differently by fluorine, chlorine, hydroxyl, cyano, carboxyl, alkoxycarbonyl having 1 to 6 carbon atoms in the alkoxy group and/or by alkylcarbonyl having 1 to 6 carbon atoms in the alkyl moiety, or
R¹ is straight-chain or branched alkenyl having 2 to 6 carbon atoms which may be mono- to trisubstituted identically or differently by fluorine, chlorine, hydroxyl, cyano, carboxyl, alkoxycarbonyl having 1 to 6 carbon atoms in the alkoxy group and/or by alkylcarbonyl having 1 to 6 carbon atoms in the alkyl moiety, or
R¹ is straight-chain or branched alkynyl having 2 to 6 carbon atoms which may be mono- to trisubstituted identically or differently by fluorine, chlorine, hydroxyl, cyano, carboxyl, alkoxycarbonyl having 1 to 6 carbon atoms in the alkoxy group and/or by alkylcarbonyl having 1 to 6 carbon atoms in the alkyl moiety, or
R¹ is benzyl which may be mono- to trisubstituted identically or differently by fluorine, chlorine, hydroxyl, cyano, carboxyl, alkoxycarbonyl having 1 to 6 carbon atoms in the alkoxy group and/or by alkylcarbonyl having 1 to 6 carbon atoms in the alkyl moiety,
R² and R³ are the same or different and are each hydrogen, fluorine, chlorine, alkyl having 1 to 6 carbon atoms, phenyl or phenoxy,
R⁴ is hydrogen or alkyl having 1 to 6 carbon atoms and
R⁵ and R⁶ are the same or different and are each hydrogen, fluorine, chlorine, alkyl having 1 to 6 carbon atoms, phenyl or phenoxy or
R⁵ and R⁶ together are a fused-on benzene ring.

3. Process according to Claim 1, **characterized in that** the starting materials used are cyclohexa-2,5-diene-1-carboxylic acid derivatives of the formula (II) where
R¹ is straight-chain or branched alkyl having 1 to 4 carbon atoms which may be mono- to trisubstituted identically or differently by fluorine, chlorine, hydroxyl, cyano, carboxyl, alkoxycarbonyl having 1 to 4 carbon atoms in the alkoxy moiety and/or by alkylcarbonyl having 1 to 4 carbon atoms in the alkyl moiety, or
R¹ is straight-chain or branched alkenyl having 2 to 4 carbon atoms which may be mono- to trisubstituted identically or differently by fluorine, chlorine, hydroxyl, cyano, carboxyl, alkoxycarbonyl having 1 to 4 carbon atoms in the alkoxy moiety and/or by alkylcarbonyl having 1 to 4 carbon atoms in the alkyl moiety, or
R¹ is straight-chain or branched alkynyl having 2 to 4 carbon atoms which may be mono- to trisubstituted identically or differently by fluorine, chlorine, hydroxyl, cyano, carboxyl, alkoxycarbonyl having 1 to 4 carbon atoms in the alkoxy moiety and/or by alkylcarbonyl having 1 to 4 carbon atoms in the alkyl moiety, or
R¹ is benzyl which may be mono- to trisubstituted identically or differently by fluorine, chlorine, hydroxyl, cyano, carboxyl, alkoxycarbonyl having 1 to 4 carbon atoms in the alkoxy moiety and/or by alkylcarbonyl having 1 to 4 carbon atoms in the alkyl moiety,
R² and R³ are each independently hydrogen, fluorine, chlorine, alkyl having 1 to 4 carbon atoms, phenyl or phenoxy,
R⁴ is hydrogen or alkyl having 1 to 4 carbon atoms and
R⁵ and R⁶ are each independently hydrogen, fluorine, chlorine, alkyl having 1 to 4 carbon atoms, phenyl or phenoxy or
R⁵ and R⁶ together are a fused-on benzene ring.

4. Process according to Claim 1, **characterized in that** sulphuric acid or chlorosulphonic acid is used as a reaction component.

5. Process according to Claim 1, **characterized in that** the working temperature is between -30°C and +50°C.

## Revendications

1. Procédé de production de benzènes substitués de formule 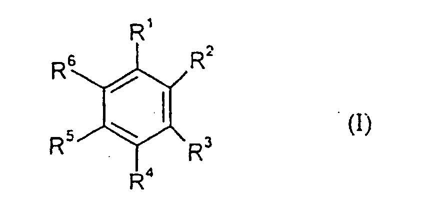 dans laquelle
R¹ est un reste alkyle portant éventuellement un substituant halogéno, hydroxy, cyano, carboxyle, alkoxycarbonyle et/ou alkylcarbonyle, un reste alcényle portant éventuellement un substituant halogéno, hydroxy, cyano, carboxyle, alkoxycarbonyle et/ou alkylcarbonyle, un reste alcynyle portant éventuellement un substituant halogéno, hydroxy, cyano, carboxyle, alkoxycarbonyle et/ou alkylcarbonyle ou un reste benzyle portant éventuellement un substituant halogéno, hydroxy, cyano, carboxyle, alkoxycarbonyle et/ou alkylcarbonyle,
R² et R³ sont identiques ou différents et représentent l'hydrogène, le fluor, le chlore, un reste alkyle, phényle ou phénoxy,
R⁴ est l'hydrogène ou un reste alkyle et
R⁵ et R⁶ sont identiques ou différents et représentent l'hydrogène, le fluor, le chlore, un reste alkyle, phényle ou phénoxy, ou bien
R⁵ et R⁶ forment ensemble un noyau benzénique condensé,
**caractérisé en ce qu'**on fait réagir des dérivés d'acide cyclohexa-2,5-diène-1-carboxylique de formule 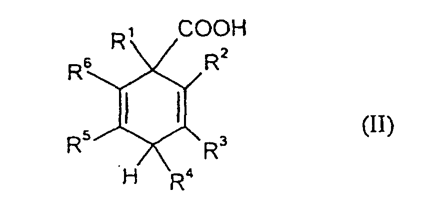 dans laquelle
R¹, R², R³, R⁴, R⁵ et R⁶ ont les définitions indiquées ci-dessus,
avec un acide, éventuellement en présence d'un diluant.

2. Procédé suivant la revendication 1, **caractérisé en ce qu'**on utilise comme composés de départ des dérivés d'acide cyclohexa-2,5-diène-1-carboxylique de formule (II), dans lesquels
R¹ représente un reste alkyle linéaire ou ramifié ayant 1 à 6 atomes de carbone, qui peut être substitué une à trois fois identiques ou différentes par un radical fluoro, chloro, hydroxy, cyano, carboxyle, alkoxycarbonyle ayant 1 à 6 atomes de carbone dans le groupe alkoxy et/ou par un radical alkylcarbonyle ayant 1 à 6 atomes de carbone dans la partie alkyle, ou bien
R¹ est un reste alcényle linéaire ou ramifié ayant 2 à 6 atomes de carbone, qui peut être substitué une à trois fois identiques ou différentes par un radical fluoro, chloro, hydroxy, cyano, carboxyle, alkoxycarbonyle ayant 1 à 6 atomes de carbone dans la partie alkoxy et/ou alkylcarbonyle ayant 1 à 6 atomes de carbone dans la partie alkyle, ou bien
R¹ représente un reste alcynyle linéaire ou ramifié ayant 2 à 6 atomes de carbone, qui peut porter un à trois substituants, identiques ou différents, fluoro, chloro, hydroxy, cyano, carboxyle, alkoxycarbonyle ayant 1 à 6 atomes de carbone dans le groupe alkoxy et/ou alkylcarbonyle ayant 1 à 6 atomes de carbone dans la partie alkyle, ou bien
R¹ est un reste benzyle qui peut porter un à trois substituants, identiques ou différents, fluoro, chloro, hydroxy, cyano, carboxyle, alkoxycarbonyle ayant 1 à 6 atomes de carbone dans la partie alkoxy et/ou alkylcarbonyle ayant 1 à 6 atomes de carbone dans la partie alkyle,
R² et R³ sont identiques ou différents et représentent l'hydrogène, le fluor, le chlore, un reste alkyle ayant 1 à 6 atomes de carbone, phényle ou phénoxy,
R⁴ représente l'hydrogène ou un reste alkyle ayant 1 à 6 atomes de carbone et
R⁵ et R⁶ sont identiques ou différents et représentent l'hydrogène, le fluor, le chlore, un reste alkyle ayant 1 à 6 atomes de carbone, phényle ou phénoxy, ou bien
R⁵ et R⁶ forment ensemble un noyau benzénique condensé.

3. Procédé suivant la revendication 1, **caractérisé en ce qu'**on utilise comme composés de départ des dérivés d'acide cyclohexa-2,5-diène-1-carboxylique de formule (II), dans lesquels
R¹ représente un reste alkyle linéaire ou ramifié ayant 1 à 4 atomes de carbone, qui peut porter un à trois substituants, identiques ou différents, fluoro, chloro, hydroxy, cyano, carboxyle, alkoxycarbonyle ayant 1 à 4 atomes de carbone dans la partie alkoxy et/ou alkylcarbonyle ayant 1 à 4 atomes de carbone dans la partie alkyle, ou bien
R¹ est un reste alcényle linéaire ou ramifié ayant 2 à 4 atomes de carbone, qui peut être substitué une à trois fois identiques ou différentes par un radical fluoro, chloro, hydroxy, cyano, carboxyle, alkoxycarbonyle ayant 1 à 4 atomes de carbone dans la partie alkoxy et/ou alkylcarbonyle ayant 1 à 4 atomes de carbone dans la partie alkyle, ou bien
R¹ est un reste alcynyle linéaire ou ramifié ayant 2 à 4 atomes de carbone, qui peut porter un à trois substituants, identiques ou différents, fluoro, chloro, hydroxy, cyano, carboxyle, alkoxycarbonyle ayant 1 à 4 atomes de carbone dans la partie alkoxy et/ou alkylcarbonyle ayant 1 à 4 atomes de carbone dans la partie alkyle, ou bien
R¹ est un reste benzyle qui peut porter un à trois substituants, identiques ou différents, fluoro, chloro, hydroxy, cyano, carboxyle, alkoxycarbonyle ayant 1 à 4 atomes de carbone dans la partie alkoxy et/ou alkylcarbonyle ayant 1 à 4 atomes de carbone dans la partie alkyle,
R² et R³ représentent, indépendamment l'un de l'autre, l'hydrogène, le fluor, le chlore, un reste alkyle ayant 1 à 4 atomes de carbone, phényle ou phénoxy,
R⁴ est l'hydrogène ou un reste alkyle ayant 1 à 4 atomes de carbone et
R⁵ et R⁶ représentent, indépendamment l'un de l'autre, l'hydrogène, le fluor, le chlore, un reste alkyle ayant 1 à 4 atomes de carbone, phényle ou phénoxy, ou bien
R⁵ et R⁶ forment ensemble un noyau benzénique condensé.

4. Procédé suivant la revendication 1, **caractérisé en ce qu'**on utilise l'acide sulfurique ou l'acide chloro-sulfonique comme composant réactionnel.

5. Procédé suivant la revendication 1, **caractérisé en ce qu'**on opère à des températures comprises entre -30°C et +50°C.
